(19) Europäisches Patentamt
European Patent Office
Office européen des brevets

(11) Veröffentlichungsnummer: **0 035 749**
**B1**

(12) # EUROPÄISCHE PATENTSCHRIFT

(45) Veröffentlichungstag der Patentschrift: **06.06.84**

(21) Anmeldenummer: **81101564.3**

(22) Anmeldetag: **05.03.81**

(51) Int. Cl.³: **C 07 D 498/04**, C 07 D 487/04, C 07 D 513/14, A 61 K 31/55 // C07D267/20, C07D281/16, C07D223/22 ,(C07D498/04, 267/00, 235/00),(C07D487/04, 235/00, 223/00),(C07D513/14, 281/00, 235/00)

(54) **Heterocyclische Verbindungen, ihre Herstellung und diese Verbindungen enthaltende Arzneimittel.**

(30) Priorität: **08.03.80 DE 3008944**

(43) Veröffentlichungstag der Anmeldung: **16.09.81 Patentblatt 81/37**

(45) Bekanntmachung des Hinweises auf die Patenterteilung: **06.06.84 Patentblatt 84/23**

(84) Benannte Vertragsstaaten: **AT BE CH DE FR IT LI LU NL SE**

(56) Entgegenhaltungen:
**DE - A - 2 248 477**
**DE - A - 2 311 019**
**DE - A - 2 548 045**

**Burger's Medicinal Chemistry, 4th Edition, 1979, Part III, pp. 509, 518-519**

**Die Akte enthält technische Angaben, die nach dem Eingang der Anmeldung eingereicht wurden und die nicht in dieser Patentschrift enthalten sind.**

(73) Patentinhaber: **C.H. BOEHRINGER SOHN, Postfach 200, D-6507 Ingelheim am Rhein (DE)**

(72) Erfinder: **Walther, Gerhard, Dr., Pfarrer-Heberer-Strasse 37, D-6530 Bingen (DE)**
Erfinder: **Schneider, Claus S., Dr., Tannenweg 1, D-6507 Ingelheim/Rhein (DE)**
Erfinder: **Weber, Karl Heinz, Dr., Kaiser-Karl-Strasse 11, D-6535 Gau-Algesheim (DE)**
Erfinder: **Fügner, Armin, Dr., Im Hippel 31, D-6535 Gau-Algesheim (DE)**

**EP 0 035 749 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft neue heterocyclische Verbindungen, ihre Herstellung nach an sich bekannten Verfahren und diese Verbindungen enthaltende Arzneimittel.

Die neuen Verbindungen entsprechen der allgemeinen Formel

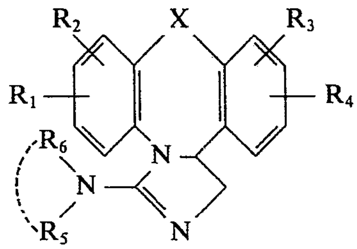

(I)

in der

$R_1$, $R_2$,
$R_3$ und $R_4$ Wasserstoff, Halogen, Alkyl- oder Alkoxy mit jeweils 1 bis 6 C-Atomen,
$R_5$ und $R_6$ Wasserstoff, Alkyl mit 1 bis 6 C-Atomen, Allyl,
$R_5$ und $R_6$ auch gemeinsam mit dem Stickstoffatom einen Pyrrolidino-, Piperidino- oder Morpholinorest bedeuten und
X für O, S oder $CH_2$ steht.

Die Verbindungen können als Racemate oder als reine Enantiomere bzw. als Gemische mit verschiedenen Anteilen der Enantiomeren vorliegen, jeweils in Form der freien Basen oder der Säureadditionssalze.

Die Reste $R_1$ bis $R_4$ bzw. $R_5$ und $R_6$ können gleich oder verschieden sein. Die Alkyl- und Alkoxygruppen können geradkettig oder verzweigt sein. Dementsprechend haben die Alkylreste folgende Formeln:

$-CH_3$, $-C_2H_5$, $-CH_2-CH_2-CH_3$, $-CH(CH_3)_2$, $-(CH_2)_3-CH_3$,

$-CH_2-CH(CH_3)_2$, $-CH(CH_3)-C_2H_5$, $-C(CH_3)_3$, $-(CH_2)_4-CH_3$,

$-CH_2-CH_2-CH(CH_3)_2$, $-CH_2-CH(CH_3)-C_2H_5$,

$-CH(CH_3)-CH_2-CH_2-CH_3$, $-CH_2-C(CH_3)_3$, $-C(CH_3)_2-C_2H_5$,

$-CH(CH_3)-CH(CH_3)_2$, $-(CH_2)_5-CH_3$, $-(CH_2)_3-CH(CH_3)_2$,

$-CH_2-CH_2-CH(CH_3)-C_2H_5$, $-CH_2-CH(CH_3)-CH_2-CH_2-CH_3$,

$-CH(CH_3)-(CH_2)_3-CH_3$, $-C(CH_3)_2-CH_2-CH_2-CH_3$,

$-CH_2-C(CH_3)_2-C_2H_5$, $-CH_2-CH_2-C(CH_3)_3$, $-CH(CH_3)-CH(CH_3)-C_2H_5$,

$-CH(CH_3)-CH_2-CH(CH_3)_2$, $-C(CH_3)_2-C(CH_3)_2$, $-CH(CH_3)-C(CH_3)_3$,

$-CH_2-CH(C_2H_5)_2$, $-CH(C_2H_5)-(CH_2)_2-CH_3$.

Die Alkoxyreste können dieselben Kohlenstoffskelette enthalten.

Als cyclische Guanidin-Derivate können die Verbindungen der Formel I (falls $R_6$ und gegebenenfalls $R_5$ Wasserstoff bedeutet) auch in der tautomeren Form

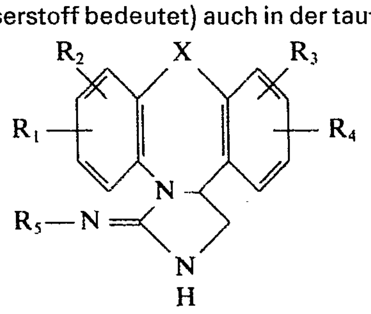

(Ia)

vorliegen.

Die Bezeichnung der Positionen ist in der nachstehenden Formel angegeben.

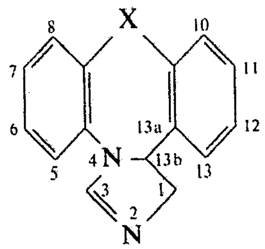

(Ib)

Zur Herstellung der Verbindungen der Formel I/Ia können folgenden Verfahren benutzt werden:

1) Umsetzung einer Verbindung der allgemeinen Formel

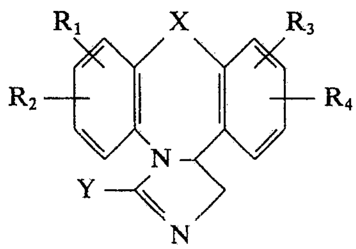

(II)

(Y gleich Halogen, z. B. Chlor, Alkoxy oder Alkylthio)

mit einem Amin der Formel

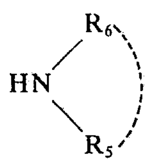

(III)

Die Umsetzung erfolgt in der Schmelze oder unter Zusatz gebräuchlicher Lösungsmittel (z. B. Alkohole, Ketone, Äther, aliphatische oder aromatische Kohlenwasserstoffe), gegebenenfalls im Autoklav.

Auch die Amine III können als Reaktionsmedium dienen. Die Umsatzung wird im allgemeinen bei Temperaturen bis zur Siedetemperatur des Lösungsmittels durchgeführt.

Die Ausgangsstoffe der Formel II können analog literaturbekannten Methoden gewonnen werden; für den Fall Y gleich Halogen sind sie z. B. durch Umsetzung von Diaminen der Formel

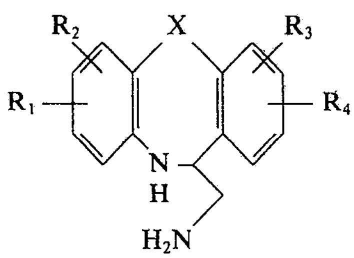

(IV)

mit bifunktionellen Kohlensäurederivaten wie Phosgen, Chlorkohlensäureestern oder N,N'-Carbonyldiimidazol zu den entsprechenden 3-Oxoverbindungen der Formel

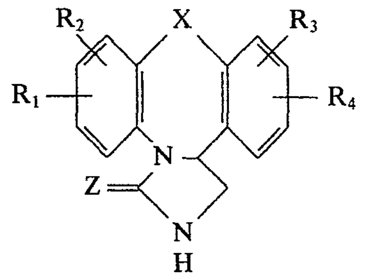

(V)

(Z gleich Sauerstoff)

und anschließende Halogenierung mit einem entsprechenden anorganischen Säurechlorid zugänglich.
Verbindungen der Formel II, in denenY eine Alkylthiogruppe bedeutet, werden z. B. durch Umsetzung der Diamine IV mit Schwefelkohlenstoff, Thiophosgen oder N,N'-Thiocarbonyldiimidazol zu den entsprechenden 3-Thioxo-Derivaten (V; Z gleich Schwefel) umgesetzt und anschließend mit einem Alkylhalogenid zu den Verbindungen II (Y gleich Alkylthio) alkyliert. Der Alkylrest in dem Substituenten Y (soweit dieser Alkoxy oder Alkylthio bedeutet) enthält vorzugsweise 1 bis 8 C-Atome und kann auch selbst substituiert sein.
Zu den Diaminen IV gelangt man nach dem Reaktionsschema:

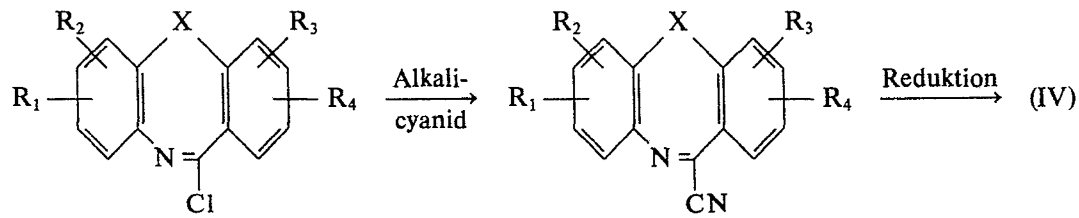


(VI) (VII)

Die Verbindungen der Formel VI sind bekannt [Lit. z. B. Helv. chim. Acta, 49/II, 1433 ff. (1966), oder Helv. chim. Acta, 50/I, 245 ff. (1967)] oder können nach bekannten Verfahren hergestellt werden. Die Umsetzung von Verbindungen der Formel VI mit einem Alkalicyanid, wie Natriumcyanid, wird vorteilhaft in DMSO bei Raumtemperatur bis 60°C durchgeführt. Die Reduktion der Cyanoverbindungen (VII) erfolgt vorzugsweise mit Lithiumaluminiumhydrid ($LiALH_4$) oder Aluminiumwasserstoff ($AlH_3$) in Tetrahydrofuran bzw. Diäthyläther.

2) Umsetzung eines Diamins der Formel IV mit einem Halogencyan, wie Bromcyan

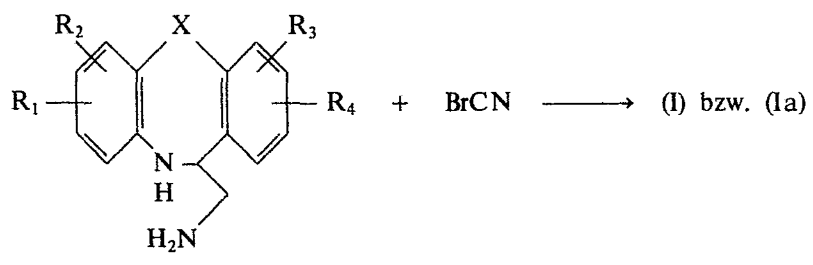


(IV)

Man erhält Verbindungen der Formel I/Ia, in denen $R_5$ und $R_6$ Wasserstoff bedeuten.
Die Umsetzung erfolgt bevorzugt bei Raumtemperatur in einem Äthanol/Tetrahydrofuran-Gemisch, kann aber auch in anderen Lösungsmitteln, wie Alkoholen, Chloroform oder Kohlenwasserstoffen wie Toluol und Xylol gegebenenfalls auch unter Zusatz einer Base (wie z. B. Kaliumcarbonat) durchgeführt werden. Die Reaktionstemperaturen sind weitgehend variabel und können bis zur Siedetemperatur des Reaktionsgemischs gehen.
Bei der Umsetzung der Verbindung IV mit z. B. Bromcyan bildet sich intermediär eine Verbindung der Formel

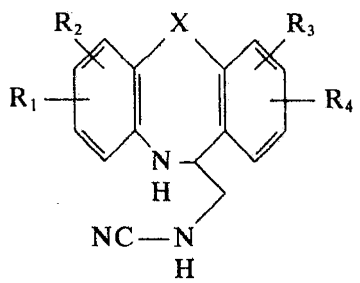

(IV a)

die somit den eigentlichen Ausgangsstoff der Ringschluß-Reaktion bildet. Es ist jedoch nicht erforderlich, diese Verbindung zu isolieren, da sie glatt zu dem gewünschten Endprodukt weiterreagiert.

3) Ringschluß einer Verbindung der Formel

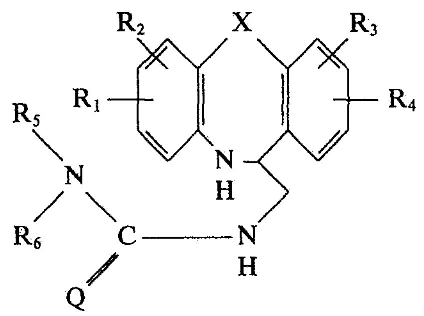

(VIII)

in der $R_1$ bis $R_6$ die obige Bedeutung haben und Q für Sauerstoff, Schwefel oder $NR_7$ steht, wobei $R_7$ Wasserstoff oder einen gegebenenfalls substituierten Alkylrest mit 1 bis 8 C-Atomen darstellt, z. B. Methyl, Äthyl, Propyl, Benzyl.
Verbindungen der Formel VIII mit Q gleich Sauerstoff oder Schwefel, $R_5$ gleich Alkyl oder Allyl und $R_6$ gleich Wasserstoff können durch Umsetzung von Diaminen der Formel IV mit Alkyl- oder Allylisocyanaten bzw. Alkyl- oder Allylisothiocyanaten erhalten werden.
Der Ringschluß zu den erfindungsmäßigen Verbindungen der Formeln I bzw. Ia erfolgt durch Einwirkung von Säurehalogeniden, wie $POCl_3$, gegebenenfalls in gebräuchlichen Lösungsmitteln, wie Toluol. Eine Variante dieses Verfahrens besteht in der S-Alkylierung der Thioharnstoffderivate VIII (Q gleich Schwefel) zu den entsprechenden Alkylthio-Verbindungen der Formel

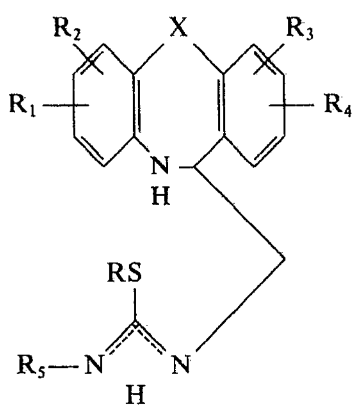

(IX)

(R gegebenenfalls substituierter Alkylrest mit 1 bis 8 C-Atomen)

die in an sich bekannter Weise durch Erwärmen in gebräuchlichen Lösungsmitteln, z. B. Alkoholen, Toluol oder in der Schmelze zu Verbindungen der Formel I bzw. Ia ringgeschlossen werden.
Verbindungen der Formel VIII mit Q gleich $NR_7$ lassen sich zum Beispiel auch durch Umsetzung von Diaminen der Formel IV mit entsprechenden substituierten oder auch unsubstituierten S-Alkyl-iso-thioharnstoffen gewinnen. Der Ringschluß zu den Verbindungen der Formel I bzw. Ia wird vorzugsweise in der Schmelze durchgeführt.

4) Umsetzung von Diaminen der Formel IV mit N,N-disubstituierten Dichlormethyleniminium-Salzen der Formel X, Reaktionsschema:

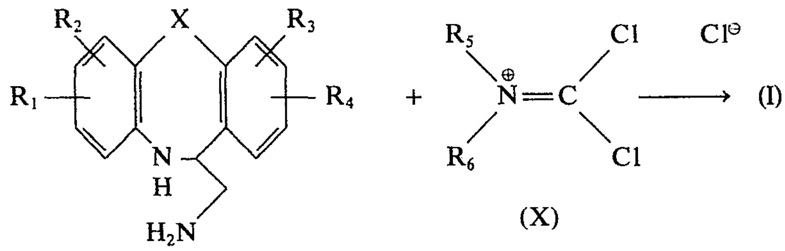

(IV) (X) (I)

Man erhält Verbindungen der Formel I mit $R_5$ und $R_6$ in der obigen Bedeutung, ausgenommen Wasserstoff. Die Reaktion wird vorteilhaft in einem inerten Lösungsmittel, z. B. Chloroform, unter Zusatz einer Base, z. B. Triäthylamin, durchgeführt.

Die nach den verschiedenen Verfahren erhaltenen Reaktionsprodukte werden mit Hilfe bekannter Labormethoden isoliert. Gegebenenfalls können die so erhaltenen Rohprodukte noch unter Verwendung besonderer Methoden, z. B. durch Säulenchromatographie, gereinigt werden, ehe man sie in Form der Base oder geeigneter Salze kristallisiert.

Gewünschtenfalls werden erfindungsgemäß erhaltene Racemate nach üblichen Methoden in die Enantiomeren aufgetrennt.

Aus primär erhaltenen Salzen werden gewünschtenfalls freie Basen, aus primär erhaltenen freien Basen Säureadditionssalze nach üblichen Methoden gewonnen.

Zur Herstellung von Säureadditionssalzen werden insbesondere solche Säuren verwendet, die zur Bildung von therapeutisch verwendbaren Salzen geeignet sind. Als solche Säuren seien beispielsweise genannt: Halogenwasserstoffsäuren; aliphatische, alicyclische, aromatische oder heterocyclische Carbonsäuren oder Sulfonsäuren, wie Essigsäure, Weinsäure, Malonsäure, Zitronensäure, Fumarsäure, Salicylsäure, Embonsäure, Methansulfonsäure, Toluolsulfonsäure; ferner Schwefelsäure, Phosphorsäure.

Die erfindungsgemäßen neuen Verbindungen sind therapeutisch verwendbar oder stellen Zwischenprodukte für die Herstellung von therapeutisch verwendbaren Verbindungen dar. Sie zeichnen sich bei relativ geringer Toxizität vor allem durch ihre lange anhaltende antiallergische, antihistaminerge und antiserotonische Wirkung aus, ferner hemmen sie die Blutblättchenaggregation. Therapeutische Anwendungsmöglichkeiten für die neuen Verbindungen sind z. B. die Behandlung von Reaktionen, die durch Freisetzung von Histamin oder Serotonin erzeugt wurden, Asthma bronchiale, allergische Bronchitis, allergische Rhinitis, allergische Konjunctivitis sowie allergische Diathesen. Von besonderer Bedeutung für therapeutische Zwecke ist die gute orale Wirkung der Verbindungen. Die orale Wirkung ist auch ein wesentlicher Vorteil gegenüber dem Dinatriumsalz der Cromoglicinsäure, einem vielbenutzten Handelsprodukt zur Behandlung von Asthma bronchiale und allergischer Bronchitis.

Aus der DE-A-2 311 019 sind Imidazolinderivate (Tetrahydro-imidazo-dibenzo-oxazepine und -thiazepine) bekannt, die den erfindungsgemäßen Verbindungen strukturell verwandt sind. Wie in der Offenlegungsschrift ausgeführt wird, zeigen die bekannten Verbindungen antidepressive Aktivität; hingegen ist ihre Antihistamin- und/oder Antiserotoninaktivität vernachlässigbar. Unerwarteterweise ist bei den Verbindungen gemäß der vorliegenden Erfindung jedoch gerade die Antihistaminwirkung besonders ausgeprägt.

Eine Gruppe Imidazolidinderivate (Tetrahydro-dibenzoimidazo-azepine) mit antihistaminischer und antiserotonischer Wirkung ist in der DE-A-2 248 477 beschrieben. Von diesen bekannten Verbindungen unterscheiden sich die erfindungsgemäßen Verbindungen dadurch, daß sie als Strukturelement eine Guanidingruppierung enthalten. Die Guanidinstruktur findet sich zwar in einzelnen $H_2$-Antihistaminika, jedoch auch in zahlreichen Arzneistoffen anderer Wirkungsrichtungen, etwa dem blutdrucksenkenden Clonidine:

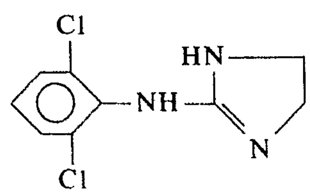

(2-[(2,6-Dichlorphenyl)amino]-2-imidazolin) oder dem antimikrobiellen Ambazone:

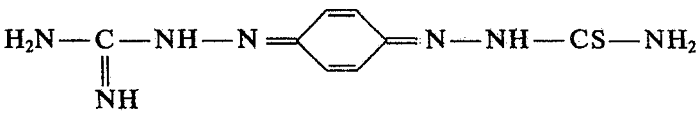

(1-Amidinohydrazono-4-thiosemicarbazono-2,5-cyclohexadien).

Dieser Situation entspricht, daß in Burger's Medicinal Chemistry, 4. Auflage, Teil III, S. 509, rechte Spalte, als Resumée der Untersuchungen an zahlreichen Guanidinderivaten gesagt wird, »that the ability to antagonize histamine was not a property of guanidines per se«. An anderer Stelle ist diesem Werk zu entnehmen (a.a.O., S. 518/519), daß die Tautomeriefähigkeit für die Guanidingruppierung gegeben sein sollte, damit sich die für Antihistaminika erwünschte flache Konformation der Guanidinstruktureinheit ergibt. Bei den erfindungsgemäßen Verbindungen mit einer disubstituierten Aminogruppe in 3-Position ist indessen Tautomerie nicht möglich. Dennoch sind diese wie die übrigen erfindungsgemäßen Verbindungen überraschenderweise stark antihistaminisch wirksam.

Für die Anwendung werden die erfindungsgemäßen Verbindungen in üblicher Weise mit Hilfs- und Trägerstoffen zu gebräuchlichen galenischen Zubereitungen verarbeitet, z. B. zu Kapseln, Tabletten, Dragées, Lösungen, Suspensionen für die orale Anwendung; zu Aerosolen für die pulmonale Gabe; zu sterilen isotonischen wäßrigen Lösungen für die parenterale Anwendung und zu Cremes, Salben, Lotionen, Emulsionen oder Sprays für die lokale Applikation.

Die Einzeldosis bei der oralen Anwendung beträgt beim Erwachsenen 0,2 bis 40, vorzugsweise 0,5 bis 10 mg. Für die Inhalation werden Einzeldosen zwischen 0,05 bis 20, vorzugsweise 0,2 bis 5 mg, appliziert, wobei übliche Zubereitungen verwendet werden, vor allem Dosieraerosole und Kapseln für die Pulverinhalation. Die angegebenen Dosen können gegebenenfalls mehrmals pro Tag verabreicht werden.

Zur Ermittlung der Wirkung der erfindungsgemäßen Verbindungen wurden diese zahlreichen pharmakologischen Prüfungen unterworfen.

(a) Versuche an allergisierten Ratten wurden durchgeführt nach passiver Sensibilisierung der Tiere mit IgE-Antikörpern und anschließender Antigenprovokation. Auf diese Weise konnte eine passive cutane Anaphylaxie (GOOSE et al. (1969), Immunology, 16, 749) (PCA) und eine passive Lungenanaphylaxie (FARMER et al. (1975), Br. J. Pharmac., 55, 57) (PLA, experimentelles Asthma) erzeugt werden.

(b) Die antianaphylaktische Wirkung wurde in Hunden, die gegenüber Spulwurmantigen eine Überempfindlichkeit der Haut zeigen (BOOTH et al. 1970), J. Lab. clin. Med., 76, 181), per os bestätigt.

(c) Antihistaminische und antiserotoninische Wirkungen:
Die Verbindungen hemmten p. o. und i. v. in Ratten, Hunden und Affen die durch intrakutane Injektion von Histamin erzeugte Histaminquaddel. Die Quantifizierung erfolgte nach Extravasation von Evans Blau-Farbstoff in die Haut durch Ausmessen der Quaddel.
Die Antiserotoninwirkung wurde durch die Aktivität gegen das Serotoninödem der Rattenpfote nachgewiesen (DOEPENER et al. (1958), Int. Arch. Allergy, 12, 89).

Als Beispiele für die Wirkung der erfindungsgemäßen Verbindungen sollen die Angaben in der nachstehenden Tabelle dienen:

| Verbindung | PCA $ED_{50}$ [mg/kg] (Ratte p. o.) | PLA $ED_{50}$ [mg/kg] (Ratte i. v.) | $LD_{50}$ [mg/kg] (Maus p. o.) |
|---|---|---|---|
| Beispiel 1 | 6 | 0,052 | 325 |
| Beispiel 1 (a) | 0,96 | — | — |
| Beispiel 1 (c) | 1,1 | — | 340 |
| Beispiel 1 (d) | 2,5 | — | — |
| Beispiel 1 (i) | 3,8 | — | — |
| Beispiel 1 (l) | 5,4 | — | — |

Nachstehend sind einige Beispiele für pharmazeutische Präparate mit erfindungsgemäßen Wirkstoffen angegeben:

## Tabletten

Zusammensetzung:

| | |
|---|---|
| Wirkstoff gemäß Formel I | 0,005 g |
| Stearinsäure | 0,001 g |
| Traubenzucker | 0,194 g |

## Inhalationsaerosol

Zusammensetzung:

| | |
|---|---|
| Wirkstoff gemäß Formel I | 1,00 Teil |
| Sojalecithin | 0,20 Teile |
| Treibgasmischung (Frigen 11, 12 und 114), ad | 100,0 Teile |

Die Zubereitung wird vorzugsweise in Aerosolbehälter mit Dosierventil abgefüllt, der einzelne Hub wird so bemessen, daß eine Dosis von 0,5 mg abgegeben wird. Für die anderen Dosierungen des angegebenen Bereichs verwendet man zweckmäßig Zubereitungen mit höherem oder niedrigerem Wirkstoffanteil.

## Kapseln für die Inhalation

Ein Wirkstoff nach Formel I wird in mikronisierter Form (Teilchengröße im wesentlichen zwischen 2 und 6 µm), gegebenenfalls unter Zusatz von mikronisierten Trägerstoffen, etwa Lactose, in Hartgelatinekapseln gefüllt. Zur Inhalation dienen übliche Geräte für die Pulverinhalation. In jede Kapsel werden z. B. zwischen 0,2 und 20 mg Wirkstoff und 0 bis 40 mg Lactose eingefüllt.

## Salbe

Zusammensetzung:

| | g/100 g Salbe |
|---|---|
| Wirkstoff gemäß der Erfindung | 2,000 |
| Rauchende Salzsäure | 0,011 |
| Natriumpyrosulfit | 0,050 |
| Gemisch aus gleichen Teilen Cetylalkohol und Stearylalkohol | 20,000 |
| Weiße Vaseline | 5,000 |
| Künstliches Bergamotteöl | 0,075 |
| Destill. Wasser, ad | 100,000 |

Die Bestandteile werden in üblicher Weise zu einer Salbe verarbeitet.

Die nachstehenden Beispiele sollen die Erfindung näher erläutern. Alle Temperaturen sind in °C gemacht.

## Beispiel 1

3-Amino-9,13b-dihydro-1H-dibenz[c,f]imidazo[1,5-a]azepin-hydrobromid

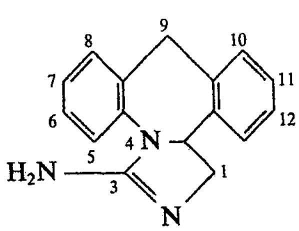

6,72 g (0,03 Mol) 6-Aminomethyl-6,11-dihydro-5H-dibenz[b,e]azepin werden in 60 ml Äthanol gelöst und eine Lösung von 3,18 g (0,03 Mol) Bromcyan in 25 ml absolutem Tetrahydrofuran unter Rühren zugetropft. Die Reaktionsmischung wird über Nacht bei Raumtemperatur gerührt, mit 50 ml Äther versetzt, und anschließend werden die Kristalle abgesaugt.

Ausbeute: 7,8 g (78,9% d. Th.); Fp.: 284—286° ($CH_3OH$/Essigester).

Die aus dem Hydrobromid mit wäßriger Natronlauge freigesetzte Base hat einen Schmelzpunkt von 205—208° (Acetonitril).

Zur Herstellung des Hydrochlorids wird eine Suspension der Base in Methanol mit der berechneten Menge ätherischer Salzsäure behandelt und anschließend das Hydrochlorid durch Zugabe von Äther ausgefällt.

Fp.: 272—273° (Methanol/Äther).

Analog Beispiel 1 können die nachfolgenden Verbindungen erhalten werden:

(a) 3-Amino-7-chlor-9,13b-dihydro-1H-dibenz[c,f]imidazo[1,5-a]azepin-hydrochlorid; Fp.: 325—329° Z. (Alkohol)
(b) 3-Amino-6-methyl-9,13b-dihydro-1H-dibenz[c,f]imidazo[1,5-a]azepin-hydrobromid;
(c) 3-Amino-1,13b-dihydro-dibenz[b,f]imidazo[1,5-d][1,4]-oxazepin-hydrobromid;

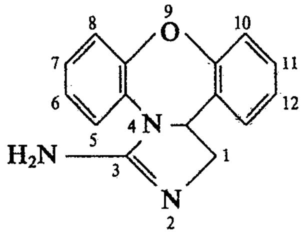

Fp.: 261—264° (Alkohol/Essigester)
(d) 3-Amino-12-chlor-1,13b-dihydro-dibenz[b,f]imidazo[1,5-d][1,4]oxazepin-hydrobromid; Fp.: 300° (Acetonitril/Essigester)
(e) 3-Amino-7-chlor-1,13b-dihydro-dibenz[b,f]imidazo[1,5-d][1,4]oxazepin-hydrobromid; Fp.: 297—300° (Alkohol/Äther)
(f) 3-Amino-6-chlor-1,13b-dihydro-dibenz[b,f]imidazo[1,5-d][1,4]oxazepin-hydrobromid; Fp.: 282—284° (Methanol/Äther)
(g) 3-Amino-6-methyl-1,13b-dihydro-dibenz[b,f]imidazo[1,5-d][1,4]oxazepin-hydrobromid; Fp.: 187—189°
(h) 3-Amino-12-methyl-1,13b-dihydro-dibenz[b,f]imidazo[1,5-d][1,4]oxazepin-hydrobromid; Fp.: 309—312°
(i) 3-Amino-1,13b-dihydro-dibenz[b,f]imidazo[1,5-d][1,4]thiazepin-hydrobromid

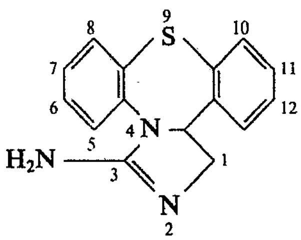

Zur Herstellung des sauren Maleinats ($C_{15}H_{13}N_3S \times C_4H_4O_4$) wird die in üblicher Weise freigesetzte Base in Methanol gelöst und mit der berechneten Menge Maleinsäure versetzt. Die ausgefallenen Kristalle werden aus Methanol unter Zusatz von Kohle umkristallisiert; Fp.: 230° Z.

(j) 3-Amino-6-chlor-1,13b-dihydro-dibenz[b,f]imidazo[1,5-d][1,4]thiazepin-hydrobromid
(k) 3-Amino-7-chlor-1,13b-dihydro-dibenz[b,f]imidazo[1,5-d][1,4]thiazepin-hydrobromid
(l) 3-Amino-12-chlor-1,13b-dihydro-dibenz[b,f]imidazo[1,5-d][1,4]thiazepin-hydrobromid; Fp.: 358—361° Z. (Methanol/Essigester)
(m) 3-Amino-6-methyl-1,13b-dihydro-dibenz[b,f]imidazo[1,5-d][1,4]thiazepin-hydrobromid
(n) 3-Amino-6-methoxy-1,13b-dihydro-dibenz[b,f]imidazo[1,5-d][1,4]thiazepin-hydrobromid

Die als Ausgangsmaterial eingesetzten Diamine der Formel IV können nach Reaktionsschema auf Seite 4 hergestellt werden; z. B. 11-Aminomethyl-7-Chlor-10,11-dihydro-dibenz[b,f][1,4]oxazepin-fumarat

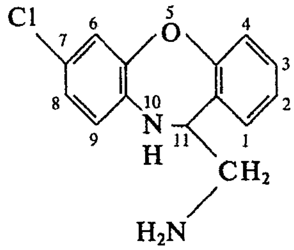

(I) 7-Chlor-11-cyano-dibenz[b,f][1,4]oxazepin

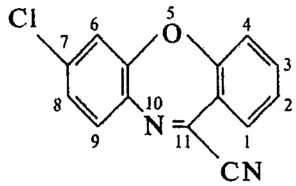

Eine Suspension von 123 g (0,47 Mol) 7,11-Dichlor-dibenz[b,f][1,4]oxazepin und 46 g (0,94 Mol) Natriumcyanid in 1400 ml Dimethylsulfoxid wird 3 Stunden bei 50—60° gerührt. Die Reaktionsmischung wird abgekühlt und auf 6 l Eiswasser gegossen. Man extrahiert mit Chloroform und dampft nach dem Trocknen mit wasserfreiem Natriumsulfat die organische Phase im Vakuum ein. Der kristalline Rückstand wird mit Methanol gewaschen und getrocknet.

Die Ausbeute beträgt 74 g (62,4% d. Th.); Fp.: 185—188°.

Analog erhält man die nachstehenden Cyanoverbindungen:

(a) 8-Chlor-11-cyano-dibenz[b,f][1,4]oxazepin;
Ausbeute: 69,7%; Fp.: 161—163° (Acetonitril)

(b) 2-Chlor-11-cyano-dibenz[b,f][1,4]oxazepin;
Ausbeute: 31,4%; der Schmelzpunkt des Rohproduktes beträgt 126—132°

(c) 11-Cyano-dibenz[b,f][1,4]oxazepin;
Ausbeute: 85,7%; Fp. des Rohproduktes 107—114°

(d) 11-Cyano-dibenz[b,f][1,4]thiazepin;

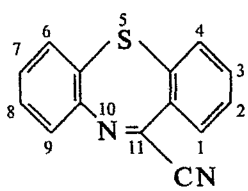

Ausbeute: 100%; Fp. des Rohproduktes 93—100°

(e) 2-Chlor-11-cyano-dibenz[b,f][1,4]thiazepin;
Ausbeute: 65,3%; Fp. des Rohproduktes 176—182°

(f) 6-Cyano-11-H-dibenz[b,e]azepin;

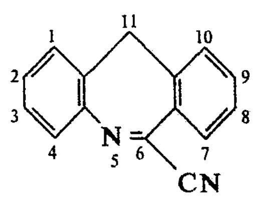

Ausbeute: 73,2%; Fp.: 98—100° (Methanol)

(g) 2-Chlor-6-cyano-11H-dibenz[b,e]azepin;
Ausbeute: 67,5%; Fp. des Rohproduktes 163—168°

(II) Reduktion von nach (I) erhaltenem 7-Chlor-11-cyano-dibenz[b,f][1,4]oxazepin

Es wird eine Lösung von Aluminiumhydrid in Tetrahydrofuran hergestellt, indem man zu einer Suspension von 7,6 g (0,2 Mol) Lithiumaluminiumhydrid in 200 ml absolutem Tetrahydrofuran unter Rühren langsam eine Lösung von 9,8 g (0,1 Mol) 100%iger Schwefelsäure in 40 ml wasserfreiem

Tetrahydrofuran zutropft. Ohne das entstehende Lithiumsulfat abzutrennen, wird innerhalb 30 min eine Lösung von 12,3 g (0,05 Mol) 7-Chlor-11-cyano-dibenz[b,f][1,4]oxazepin in 80 ml absolutem Tetrahydrofuran zugegeben. Die Reaktionsmischung wird noch 2 h bei Raumtemperatur gerührt, und anschließend wird unter Eiskühlung der Überschuß des Hydrids durch Zugabe von 25 ml Wasser zersetzt. Die anorganischen Bestandteile werden abgesaugt und zweimal mit Chloroform ausgeführt. Die Tetrahydrofuran- und die Chloroform-Lösungen werden vereinigt, mit wäßriger Kochsalzlösung gewaschen und nach dem Trocknen mit Natriumsulfat im Vakuum eingeengt. Als Rückstand bleiben 25 g dunkelrotes Öl zurück. Zur Reinigung wird das Öl in Methanol gelöst und mit der berechneten Menge Fumarsäure in das sauere Fumarat überführt.

Ausbeute: 26 g (71,3% d. Th.), Fp.: 228° Z.

Nach dem unter (II) beschriebenen Verfahren wurden die folgenden Diamine hergestellt:

(a) 11-Aminomethyl-8-chlor-10,11-dihydro-dibenz[b,f][1,4]oxazepin-fumarat.
Ausbeute: 54,6%. Fp. des Rohproduktes 239—241° Z.

(b) 11-Aminomethyl-2-chlor-10,11-dihydro-dibenz[b,f][1,4]oxazepin-fumarat.
Ausbeute: 54,4%. Fp. des Rohproduktes 228—229° Z. Die Base hat einen Schmelzpunkt von 131—133° (Acetonitril).

(c) 11-Aminomethyl-10,11-dihydro-dibenz[b,f][1,4]oxazepin.
Ausbeute: 65,5%. Der Schmelzpunkt der Base beträgt 119—122° (Acetonitril).

(d) 11-Aminomethyl-10,11-dihydro-dibenz[b,f][1,4]thiazepin-fumarat.

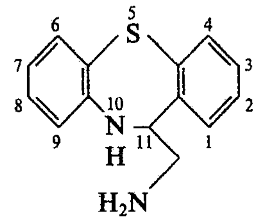

Ausbeute: 61%. Fp. des Rohproduktes 204—206 Z.

(e) 11-Aminomethyl-28-chlor-10,11-dihydro-dibenz[b,f][1,4]thiazepin-fumarat.
Ausbeute: 68,8%. Fp. des Rohproduktes 202° Z.

(f) 6-Aminomethyl-6,11-dihydro-5H-dibenz[b,e]azepin-fumarat.

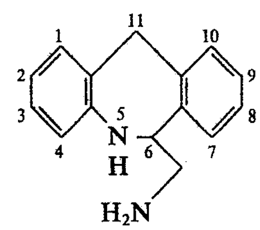

Ausbeute: 72,3%. Fp. des Rohproduktes 192—193° Z.

(g) 6-Aminomethyl-2-chlor-6,11-dihydro-5H-dibenz[b,e]azepin-fumarat.
Ausbeute: 42,1%. Fp. des Rohproduktes 197—198° Z.

Beispiel 2

3-Morpholino-1,13b-dihydro-dibenz[b,f]imidazo[1,5-d][1,4]oxazepin-fumarat

(I) 3-Methylmercapto-1,13b-dihydro-dibenz[b,f]imidazo[1,5-d][1,4]oxazepin-hydrojodid

20 g (0,075 Mol) 1,2,3,13b-Tetrahydro-dibenz[b,f]imidazo[1,5-d][1,4]oxazepin-3-thion (Fp.: 199—201°; dargestellt aus 11-Aminomethyl-10,11-dihydro-dibenz[b,f][1,4]oxazepin und Schwefelkohlenstoff) werden mit einer Lösung von 21,4 g (0,15 Mol) Methyljodid in 170 ml Methanol versetzt und das Gemisch drei Stunden unter Rückfluß erhitzt. Hierbei erhält man zunächst eine klare Lösung, aus der sich allmählich Kristalle abscheiden. Nach dem Abkühlen wird der Niederschlag abgesaugt und getrocknet.

Rohausbeute: 26,6 g (87% d. Th.); Fp.: 219—228° Z.

In analoger Weise erhält man z. B. ausgehend von 6-Aminomethyl-6,11-dihydro-5H-dibenz[b,e]azepin über 2,3,9,13b-Tetrahydro-1H-dibenz[c,f]imidazo[1,5-a]azepin-3-thion (Fp.: 222—224°) 3-Methylmercapto-9,13b-dihydro-1H-dibenz[c,f]imidazo[1,5-a]azepin-hydrojodid (Fp. des Rohproduktes 274—283° Z.).

Die 3-Methylmercapto-Derivate können ohne weitere Reinigung weiter umgesetzt werden.

(II) 8,2 g (0,02 Mol) 3-Methylmercapto-1,13b-dihydro-dibenz[b,f]imidazo[1,5-d][1,4]oxazepin-hydrojodid werden mit 25 ml Morpholin unter Rühren zwei Stunden unter Rückfluß erhitzt. Die hellgelbe Lösung wird im Vakuum eingedampft und der Rückstand zwischen Äther und 2 N-Natronlauge verteilt. Die organische Phase wird anschließend mit Wasser gewaschen, mit Natriumsulfat getrocknet und eingeengt. Der Rückstand wird aus Acetonitril umkristallisiert (Fp.: 141—142°). Zur Darstellung des sauren Fumarats wird die so erhaltene Base in Alkohol gelöst und mit der berechneten Menge Fumarsäure versetzt. Beim Abkühlen erhält man 5,8 g (66,4% d. Th.) 3-Morpholino-1,13b-dihydro-dibenz[b,f]imidazo[1,5-d][1,4]oxazepin-hydrogenfumarat.
Fp.: 189—191° Z.

Ausgehend von den entsprechenden Methylmercapto-Verbindungen können analog (II) die nachstehenden Verbindungen dargestellt werden:

(a) 3-n-Pentylamino-9,13b-dihydro-1H-dibenz[c,f]imidazo[1,5-a]azepin, das in üblicher Weise in das Hydrochlorid (Fp.: 192—195°) überführt wird.
(b) 3-Morpholino-9,13b-dihydro-1H-dibenz[c,f]imidazo[1,5-a]azepin-hydrochlorid; Fp.: 204—207° Z.

Beispiel 3

3-Allylamino-9,13b-dihydro-dibenz[b,f]imidazo[1,5-d][1,4]oxazepin-hydrochlorid

8,2 g (0,02 Mol) 3-Methylmercapto-1,13b-dihydro-dibenz[b,f]imidazo[1,5-d][1,4]oxazepin-hydrojodid weerden mit 3,42 g (0,06 Mol) Allylamin zwei Stunden unter Rühren auf 70° erhitzt. Nach dem Abkühlen wird das hellgelbe Öl mit 2 N-Natronlauge versetzt und anschließend mit Äther extrahiert. Die organische Phase wird abgetrennt, mit Wasser gewaschen und über wasserfreiem Natriumsulfat getrocknet. Beim Einengen im Vakuum erhält man 5,6 g Rohbase, die in üblicher Weise mit methanolischer Salzsäure in das Hydrochlorid überführt wird.

Ausbeute: 4,1 g (62,6% d. Th.); Fp.: 189—192° (Acetonitril).

Beispiel 4

3-Dimethylamino-1,13b-dihydro-dibenz[b,f]imidazo[1,5-d][1,4]oxazepin-hydrochlorid

3 g (7,4 mMol) 3-Methylmercapto-1,13b-dihydro-dibenz[b,f]imidazo[1,5-d][1,4]oxazepin-hydrojodid werden mit einer Lösung von 15 ml Dimethylamin in 30 ml Äthanol drei Stunden in einem Autoklav auf 130° erhitzt. Das nach dem Eindampfen der Reaktionslösung zurückbleibende Harz wird mit Chloroform aufgenommen und mit 2 n-Natronlauge ausgeschüttelt. Die organische Phase wird mit Natriumsulfat getrocknet und das Lösungsmittel im Vakuum abdestilliert. Man erhält 2,6 g Öl (Rohbase), das mit methanolischer Salzsäure in das Hydrochlorid (Fp.: 163—167°; Acetonitril/Äther) überführt wird. Die Substanz enthält ein Mol Wasser (Karl Fischer-Titration).

Nach dem im Beispiel 4 beschriebenen Verfahren werden aus den entsprechenden Methylmercapto-Derivaten die nachstehenden Verbindungen hergestellt:

(a) 3-Dimethylamino-9,13b-dihydro-1H-dibenz[c,f]imidazo[1,5-a]azepin-hydrochlorid; Fp.: 268—271°
(b) 3-Dimethylamino-1,13b-dihydro-dibenz[b,f]imidazo[1,5-d][1,4]thiazepin-hydrochlorid; Fp.: 241—244° (die Substanz enthält 0,5 Mol Wasser).

Unter den gleichen Reaktionsbedingungen erhält man durch Umsetzung von 3-Methylmercapto-9,11b-dihydro-1H-dibenz[c,f]imidazo[1,5-a]azepin-hydrojodid mit alkoholischen Lösungen von Ammoniak bzw. den entsprechenden Aminen die folgenden Verbindungen:

(c) 3-Amino-9,13b-dihydro-1H-dibenz[c,f]imidazo[1,5-a]azepin-hydrochlorid; Fp.: 283—286°. Die Substanz ist mit dem nach Beispiel 1 erhaltenen Produkt identisch.
(d) 3-Pyrrolidino-9,13b-dihydro-1H-dibenz[c,f]imidazo[1,5-a]azepin-hydrochlorid; Fp.: 158—162° Z. (Alkohol/Äther). Die Substanz enthält 0,25 Mol Wasser.
(e) 3-Methylamino-9,13b-dihydro-1H-dibenz[c,f]imidazo[1,5-a]azepin-hydrochlorid; Fp.: 234° Z. (Methanol/Äther).
(f) 3-Äthylamino-9,13b-dihydro-1H-dibenz[c,f]imidazo[1,5-a]azepin-hydrochlorid; Fp.: 275—280°.

(g) 3-Allylamino-1,13b-dihydro-1H-dibenz[c,f]imidazo[1,5-a]azepin-hydrochlorid; Fp.: 202—205° (Acetonitril/Essigester).

Beispiel 5

3-Dimethylamino-7-chlor-9,13b-dihydro-1H-dibenz[c,f]imidazo[1,5-a]azepin-hydrochlorid

In eine Lösung von 6,7 g (0,026 Mol 6-Aminomethyl-2-chlor-6,11-dihydro-5H-dibenz[b,e]azepin und 5,76 g (0,057 Mol) Triäthylamin in 70 ml Chloroform werden unter Rühren 4,21 g (0,026 Mol) Dichlormethylendimethylimmoniumchlorid eingetragen. Hierbei tritt eine leicht exotherme Reaktion ein. Die Reaktionslösung wird noch eine Stunde unter Rückfluß erhitzt, abgekühlt und nacheinander mit 2 N-Salzsäure und 2 N-Natronlauge ausgeschüttelt. Die organische Phase wird mit Wasser gewaschen, über wasserfreiem Natriumsulfat getrocknet und im Vakuum eingedampft. Der Rückstand wird anschließend an Kieselgel chromatographiert. Zunächst werden hierbei mit Aceton und Methanol Verunreinigungen abgetrennt und dann das gewünschte Produkt mit einem Gemisch von Chloroform/Methanol conc. $NH_3$ : 65/35/1 eluiert. Beim Eindampfen des Chloroform/Methanol/conc. $NH_3$-Eluates erhält man 4,5 g Öl. Diese Rohbase wird in Äther gelöst und nach dem Abfiltrieren von unlöslichen Anteilen mit methanolischer Salzsäure in das Hydrochlorid überführt.

Ausbeute: 3,7 g (40% d. Th.). Fp.: 195—197° Z. aus Acetonitril/Essigester. Die Substanz enthält 0,5 Mol Wasser.

Beispiel 6

3-Methylamino-6-chlor-9,13b-dihydro-dibenz[b,f]imidazo[1,5-d]oxazepin-hydrochlorid

(I) Eine Lösung von 9,2 g (0,035 Mol) 11-Aminomethyl-8-chlor-10,11-dihydro-dibenz[b,f][1,4]oxazepin und 2,58 g (0,035 Mol) Methylisothiocyanat wird zwei Stunden bei Raumtemperatur gerührt und anschließend über Nacht stehengelassen. Beim Einengen der Reaktionslösung erhält man einen kristallinen Rückstand von 11,7 g (Fp.: 126—132°).

(II) 10 g (0,03 Mol) des so erhaltenen Thioharnstoff-Derivates werden in einer methanolischen Lösung von 6,38 g (0,045 Mol) Methyljodid in 80 ml Methanol gelöst, und anschließend wird die Reaktionsmischung zwei Stunden unter Rückfluß erhitzt. Das ausgefallene N-{8-Chlor-10,11-dihydro-dibenz[b,f][1,4]oxazepin-11-yl}methyl-S-methyl-thioharnstoff-hydrojodid (Fp.: 212—215°) wird abgesaugt und getrocknet.

(III) Zur Freisetzung der Base wird eine Suspension von 9,7 g des nach (II) erhaltenen Hydrojodids in Wasser mit 2 N-Natronlage und Äther versetzt und so lange gerührt, bis zwei klare Schichten entstehen. Die organische Phase wird abgetrennt, mit wasserfreiem Natriumsulfat getrocknet und im Vakuum eingedampft. Der Rückstand von 7,1 g (0,02 Mol Base) wird in 60 ml Xylol gelöst und die Lösung 11 Stunden unter Rückfluß erhitzt. Die beim Abkühlen der Reaktionslösung anfallenden Kristalle (5,6 g) werden abgesaugt, in Methanol gelöst und mit ätherischer Salzsäure in das Hydrochlorid überführt. Zur weiteren Reinigung wird das Salz in verdünnter Natriumacetat-Lösung aufgenommen. Die hierbei entstehende wäßrige Lösung wird zweimal mit Äther ausgeschüttelt, mit Natronlauge alkalisch gestellt und anschließend mit Chloroform extrahiert. Die organische Phase wird mit wasserfreiem Natriumsulfat getrocknet und im Vakuum eingedampft. Die zurückbleibende Base wird in das Hydrochlorid überführt.

Ausbeute: 4,1 g (61% d. Th.); Fp.: 297—300° ($CH_3OH$/Äther)

Ausgehend von 11-Aminomethyl-10,11-dihydro-dibenz[b,f][1,4]oxazepin erhält man analog (I) und (II) N{10,11-Dihydro-dibenz[b,f][1,4]oxazepin-11-yl}methyl-S-methyl-thioharnstoff-hydrojodid. Die hieraus freigesetzte Base wird durch dreistündiges Erhitzen in Toluol unter Abspaltung von Methylmercaptan zu 3-Methylamino-9,13b-dihydro-dibenz[b,f]imidazo[1,5-d][1,4]oxazepin umgesetzt. Das Hydrochlorid hat einen Schmelzpunkt von 328—330° Z.

Beispiel 7

3-Methylamino-7-chlor-9,13b-dihydro-dibenz[b,f]imidazo[1,5-d][1,4]oxazepin-hydrochlorid

(I) N-{7-Chlor-10,11-dihydro-dibenz[b,f][1,4]oxazepin-11-yl}-N-methyl-harnstoff

Zu einer Lösung von 11 g (0,042 Mol) 11-Aminomethyl-7-chlor-10,11-dihydro-dibenz[b,f][1,4]oxazepin in 60 ml Toluol wird unter Rühren eine Lösung von 2,68 g (0,045 Mol) Methylisocyanat in 40 ml Toluol zugetropft. Nach einer Reaktionszeit von 2 Stunden werden die ausgefallenen Kristalle abgesaugt und getrocknet.
Ausbeute: 10,8 g (80,5% d. Th.); Fp.: 146—149°.

(II) Eine Suspension von 10,8 g (0,034 Mol) des nach (I) hergestellten Harnstoffderivates in 200 ml Toluol wird mit 10,8 ml Phosphoroxychlorid versetzt und die Reaktionsmischung 30 Minuten unter Rückfluß erhitzt. Nach dem Abdampfen des Lösungsmittels verteilt man den Rückstand zwischen kalter verdünnter Natronlauge und Chloroform. Die organische Phase wird über wasserfreiem Natriumsulfat getrocknet und im Vakuum eingeengt.
Ausbeute: 6,2 g (60,9% d. Th.); Fp.: 163—165° (Acetonitril). Das in üblicher Weise dargestellte Hydrochlorid hat einen Schmelzpunkt 298—302° ($CH_3OH$/Essigester).

Ausgehend von 6-Aminomethyl-6,11-dihydro-5H-dibenz[b,e]azepin erhält man durch Umsetzung von Isopropylisocyanat das entsprechende Harnstoff-Derivat (Fp.: 190—192°), das analog b) in 3-Isopropylamino-9,13b-dihydro-1H-dibenz[c,f]imidazo[1,5-a]azepin umgewandelt wird.
Das Hydrochlorid schmilzt bei 241—243° unter Zersetzung.

Beispiel 8

(−)3-Amino-9,13b-dihydro-1H-dibenz[c,f]imidazo[1,5-a]azepin-hydrochlorid bzw.
(+)3-Amino-9,13b-dihydro-1H-dibenz[c,f]imidazo[1,5-a]azepin-hydrochlorid

36,5 g (0,146 Mol) racemische 3-Amino-9,13b-dihydro-1H-dibenz[c,f]imidazo[1,5-a]azepin-Base und 55,1 g (0,164 Mol) Dibenzoyl-L(+)weinsäure werden in 1 Liter Methanol heiß gelöst. Beim Abkühlen bildet sich ein Niederschlag, der nach längerem Stehen abgesaugt wird. Die anfallenden Kristalle werden bis zu konstantem Schmelzpunkt und Drehwert aus Methanol umkristallisiert.
Fp.: 150—152° Z.; $[\alpha]_D^{25}$ −200° (c=1; Methanol).
Die in üblicher Weise freigesetzte Base wird in Methanol gelöst und mit ätherischer Salzsäure in das Hydrochlorid überführt.
Fp.: 266—269°; $[\alpha]_D^{25}$ −285° (c=1; Alkohol).
Ganz analog erhält man unter Verwendung von Dibenzoyl-D(−)weinsäure das (+)Enantiomere.
Hydrochlorid: Fp.: 266—269°; $[\alpha]_D^{25}$ +285° (c=1; Alkohol).

**Patentansprüche**

1. Verbindungen der allgemeinen Formel

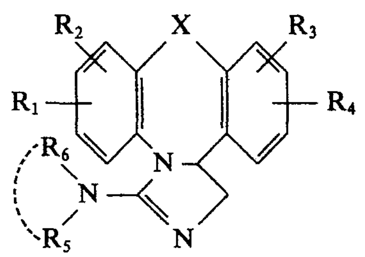

(I)

in der

$R_1$, $R_2$,
$R_3$ und $R_4$ Wasserstoff, Halogen, Alkyl- oder Alkoxy mit jeweils 1 bis 6 C-Atomen,
$R_5$ und $R_6$ Wasserstoff, Alkyl mit 1—6 C-Atomen, Allyl,
$R_5$ und $R_6$ auch gemeinsam mit dem Stickstoffatom einen Pyrrolidino-, Piperidino- oder Morpholinorest bedeutet und
X für O, S oder $CH_2$ steht,

in Form der Racemate und der Enantiomeren bzw. Gemische von Enantiomeren, jeweils als freie Basen oder als Säureadditionssalze.

2. Verbindungen nach Anspruch 1, in denen $R_5$ und $R_6$ Wasserstoff bedeuten.

3. Arzneimittel, gekennzeichnet durch einen Gehalt an einer oder mehreren Verbindungen nach Anspruch 1 neben üblichen Hilfsstoffen.

4. Verbindungen nach Anspruch 1 zur Verwendung bei der Behandlung von Asthma bronchiale und allergischer Bronchitis.

5. Verfahren zur Herstellung von Verbindungen nach Anspruch 1, dadurch gekennzeichnet, daß man

a) eine Verbindung der allgemeinen Formel

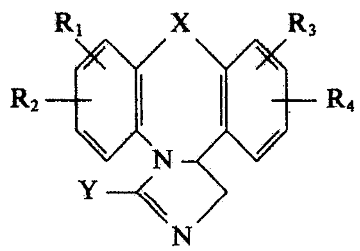

(II)

(Y gleich Halogen, Alkoxy oder Alkylthio)

mit einem Amin der Formel

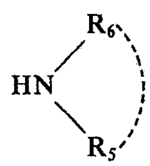

(III)

unter Zusatz eines Lösungsmittels oder in der Schmelze umsetzt oder daß man

b) zur Herstellung von Verbindungen der Formel I, in denen $R_5$ und $R_6$ Wasserstoff bedeuten, bei einem Diamin der allgemeinen Formel

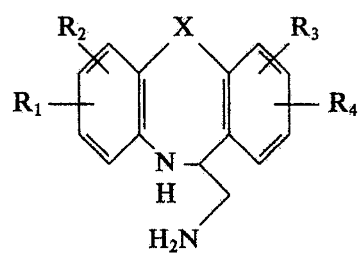

(IV)

mit einem Halogencyan bzw. bei der intermediär entstehenden Verbindung der allgemeinen Formel

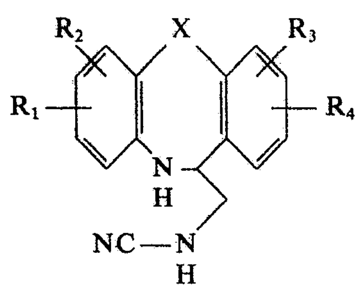

(IV a)

durch intramolekulare Umsetzung einen Ringschluß durchführt oder daß man

c) bei einer Verbindung der allgemeinen Formel

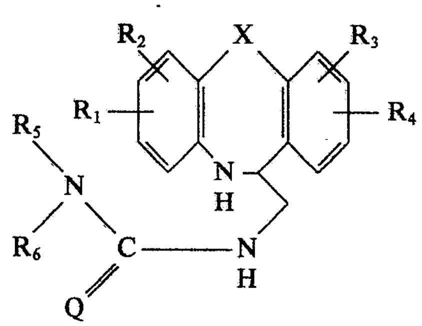

(VIII)

in der $R_1$ bis $R_6$ die obige Bedeutung haben und Q für Sauerstoff, Schwefel oder $NR_7$ steht, wobei $R_7$ H oder einen gegebenenfalls substituierten Alkylrest mit 1—8 C-Atomen darstellt, durch Einwirkung von Säurehalogeniden wie $POCl_3$ einen Ringschluß vornimmt bzw. daß man im Falle Q gleich S und $R_6$ gleich Wasserstoff die entsprechende Verbindung der Formel VIII zunächst zu dem Isothioharnstoff der allgemeinen Formel

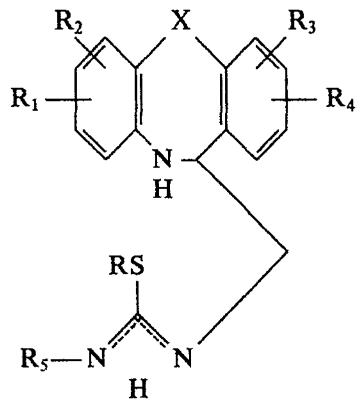

(IX)

(R gegebenenfalls substituierter Alkylrest mit 1—8 C-Atomen)

alkyliert und diesen durch Erwärmen in inerten Lösungsmitteln oder in der Schmelze einem Ringschluß unterwirft oder daß man

d) ein Diamin der allgemeine Formel IV mit N,N-disubstituierten Dichlormethyleniminiumsalzen der allgemeinen Formel

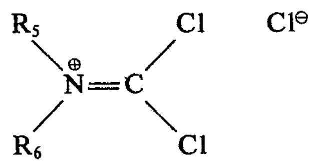

(X)

umsetzt

und daß man gewünschtenfalls die erhaltenen Racemate in die Enantiomeren aufspaltet und/oder aus primär erhaltenen freien Basen Säureadditionssalze, aus primär erhaltenen Säureadditionssalzen freie Basen herstellt.

**Claims**

1. Compounds of general formula

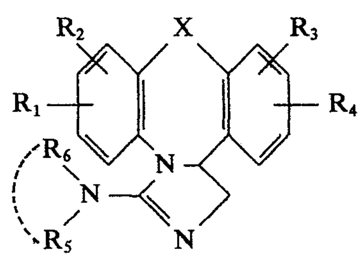

(I)

wherein

$R_1$, $R_2$,

$R_3$ and $R_4$ represent hydrogen, halogen, alkyl or alkoxy, each with 1 to 6 carbon atoms,

$R_5$ and $R_6$ represent hydrogen, alkyl with 1 to 6 carbon atoms, allyl,

$R_5$ and $R_6$ also represent, together with the nitrogen atom, a pyrrolidino, piperidino or morpholino group and

X represents O, S or $CH_2$,

in the form of the racemates and the enantiomers or mixtures of enantiomers, occuring as free bases or as acid addition salts.

2. Compounds as claimed in claim 1 wherein $R_5$ and $R_6$ represent hydrogen.

3. Pharmaceutical compositions, characterised in that they contain one or more compounds as claimed in claim1, together with conventional excipients.

4. Compounds as claimed in claim 1 for use in the treatment of bronchial asthma and allergic bronchitis.

5. Process for preparing compounds as claimed in claim 1, characterised in that

a) a compound of general formula

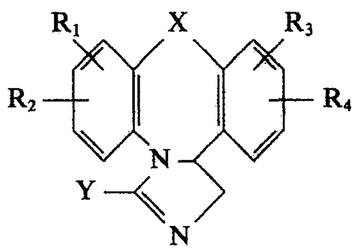

(II)

(wherein Y represents halogen, alkoxy or alkylthio)

is reacted with an amine of formula

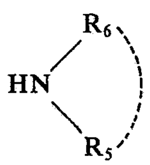

(III)

with the addition of a solvent or in the melt, or

b) to prepare compounds of formula 1 wherein $R_5$ and $R_6$ represent hydrogen, cyclisation is effected in a diamine of general formula

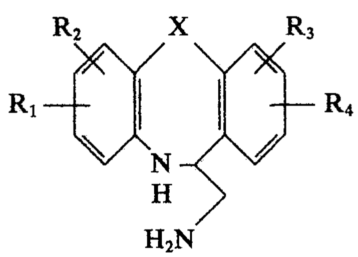

(IV)

with a cyanogen halide or in the intermediately formed compound of general formula

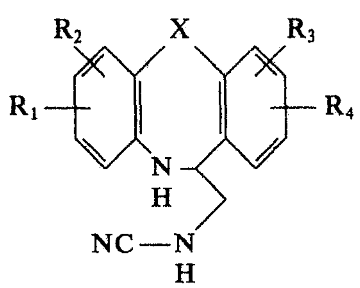

(IV a)

by intramolecular reaction, or

c) in a compound of general formula

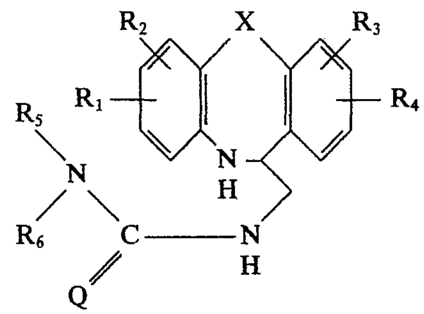

(VIII)

wherein $R_1$ to $R_6$ are as hereinbefore defined and Q represents oxygen, sulphur or $NR_7$, wherein $R_7$ represents H or an optionally substituted alkyl group with 1 to 8 carbon atoms, cyclization is effected by the action of acid halides such as $POCl_3$ or where Q represents S and $R_6$ represents hydrogen, the corresponding formula VIII is first alkylated to form the isiothiourea of general formula

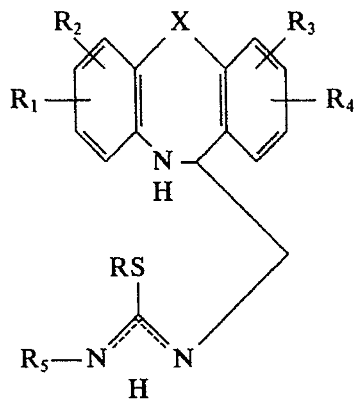

(IX)

(wherein R represents an optionally substituted alkyl group with 1—8 carbon atoms)
and this is then subjected to cyclization, by heating in an inert solvent or in the melt, or

d) a diamine of general formula IV is reacted with N,N-disubstituted dichloromethylene-iminium salts of general formula

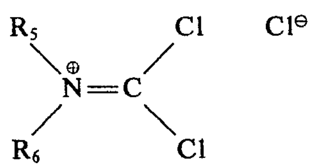

(X)

and if desired, the racemates obtained are resolved into the enantiomers and/or acid addition salts are prepared from free bases obtained primarily, and free bases are prepared from acid addition salts obtained primarily.

**Revendications**

1. Composés de formule générale

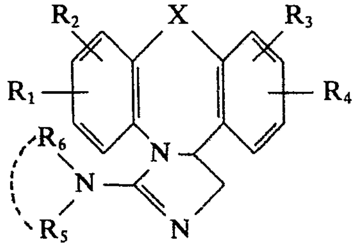

(I)

dans laquelle

$R_1$, $R_2$,
$R_3$ et $R_4$ représentent hydrogène, halogène, alcoyle ou alcoxy avec à chaque fois 1 à 6 atomes de C,
$R_5$ et $R_6$ représentent hydrogène, alcoyle avec 1—6 atomes C, allyle,
$R_5$ et $R_6$ représentent également ensemble avec l'atome d'azote un radical pyrrolidino, pipéridino ou morpholino et
X remplace O, S ou $CH_2$,
sous forme du racémate ou des énantiomères ou respectivement de mélanges d'énantiomères à chaque fois sous forme de bases libres ou sous forme de sels d'addition d'acides.

2. Composés selon la revendication 1, dans lesquels $R_5$ et $R_6$ représentent l'hydrogène.

3. Médicaments, caractérisés par une teneur en un ou plusieurs composés selon la revendication 1 conjointement à des adjuvants usuels.

4. Composés selon la revendication 1, pour l'utilisation dans le traitement de l'asthme bronchique et de la bronchite allergique.

5. Procédé de préparation de composés selon la revendication 1, caractérisé en ce que

a) on fait réagir un composé de formule générale

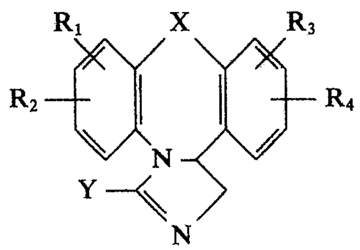

(II)

(Y tel qu'halogène, alcoxy ou alcoylthio)
avec une amine de formule

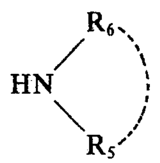

(III)

en présence d'un solvant ou à l'état fondu ou en ce que

b) pour la préparation de composés de formule I dans lesquels $R_5$ et $R_6$ représentent l'hydrogène, on effectue une fermeture de cycle dans une diamine de formule générale

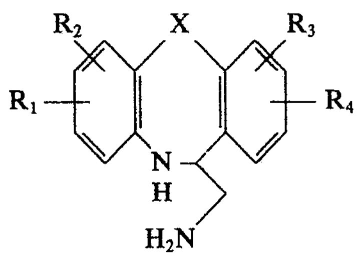

(IV)

au moyen d'un halogénure de cyanogène ou respectivement dans le cas du composé résultant de façon intermédiaire de formule générale

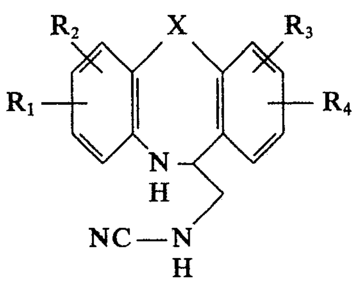

(IVa)

par réaction intramoléculaire ou en ce que

c) dans le cas d'un composé de formule générale

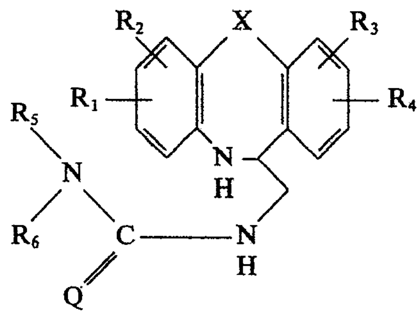

(VIII)

dans laquelle $R_1$ à $R_6$ ont la signification indiquée plus haut et Q remplace l'oxygène, le soufre ou $NR_7$, $R_7$ représentant H ou un radical alcoyle avec 1—8 atomes de C éventuellement substitué, on effectue une fermenture de cycle par action d'halogénures d'acide tels que $POCl_3$ ou respectivement en ce que dans le cas où Q est tel que S et $R_6$ est tel qu'hydrogène on alcoyle le composé correspondant de formule VIII d'abord pour donner l'isothiourée de formule général

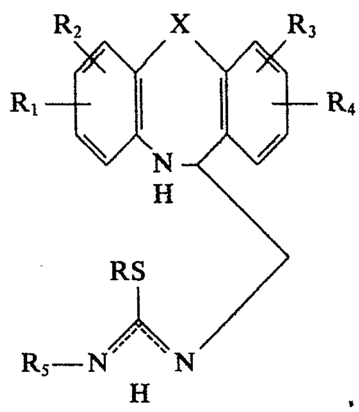

(IX)

(R radical alcoyle avec 1—8 atomes de C éventuellement substitué)

et on soumet ce dernier à une fermeture de cycle par chauffage dans des solvants inertes ou à l'état fondu ou en ce que

d) on fait réagir une diamine de formule générale IV avec des sels de dichlorométhylèneiminium N,N-disubstitués de formule générale

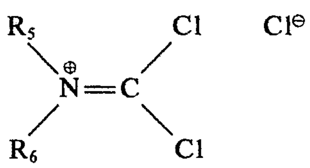

(X)

et en ce qu'on clive si on le désire les racémates obtenus en les énantiomères et/ou on prépare les sels d'addition d'acides à partir des bases obtenues au départ, ou les bases libres à partir des sels d'addition d'acides obtenus au départ.